# FASCICULE DE BREVET EUROPEEN

(11) **EP 0 742 192 B1**
(45) Date de publication et mention de la délivrance du brevet: **22.12.1999**
(21) Numéro de dépôt: 96400975.7
(22) Date de dépôt: 07.05.1996
(51) Int. Cl.: C07C 17/395, C07C 19/08

(54) **Purification du pentafluoroéthane**
Reinigung von Pentafluorethan
Purification of pentafluoroethane

(30) Priorité: 10.05.1995 FR 9505519
(43) Date de publication de la demande: 13.11.1996
(73) Titulaire: ELF ATOCHEM S.A., 92800 Puteaux, Hauts-de-Seine (FR)
(72) Inventeur: Hub, Serge, 69100 Villeurbanne (FR); Ravenel, Pierre, 69230 Saint-Genis Laval (FR)
(74) Mandataire: Leboulenger, Jean

(56) Documents cités:
- EP-A- 0 506 525
- EP-A- 0 687 660
- WO-A-94/02439
- US-A- 4 319 060

## Description

La présente invention concerne le domaine des hydrocarbures fluorés et a plus particulièrement pour objet la purification du pentafluoroéthane (ci-après F125) contenant du chloropentafluoroéthane (F115) par hydrogénolyse catalytique en phase vapeur.

Le F125 est un H.F.C. (HydroFluoroCarbone) qui peut être utilisé comme substitut au F115 (un CFC ou ChloroFluoroCarbone) dans le domaine de la réfrigération basse température.

Les procédés connus pour l'obtention du F125 comprennent entre autres la fluoration du perchloréthylène ou de ses dérivés fluorés comme le 1,1-difluoro-1,2,2-trichloroéthane (F122), le 1,1-dichloro-2,2,2-trifluoroéthane (F123) et le 1-chloro-1,2,2,2-tétrafluoroéthane (F124), la fluoration du chlorotrifluoroéthylène (F1113) ou la réduction chimique du F115, notamment l'hydrogénolyse de ce dernier qui est enseignée par la demande de brevet EP 506 525. Dans la majorité des cas, ces voies de synthese du F125 conduisent à un F125 brut qui est pollué par des quantités non négligeables de F115, soit par formation de sous-produits due aux températures élevées nécessaires à l'obtention de rendements élevés en F125 (cas des réactions de fluoration), soit en raison de conversions non quantitatives du produit de départ (cas de l'hydrogénolyse du F115).

Comme indiqué dans le brevet US 5 087 329, la séparation des composés F125 et F115 par distillation est très difficile, voire impossible si l'on souhaite obtenir une teneur très faible en F115 résiduel. On recherche donc actuellement des procédés de purification du F125 permettant d'abaisser les teneurs en F115 dans le F125 produit.

Plusieurs procédés de purification du F125 sont déjà décrits dans l'art antérieur :
- le brevet US 5 087 329 précité fait appel à une distillation extractive consistant, avant distillation, à ajouter au mélange F125-F115 un agent d'extraction constitué par un hydrocarbure fluoré en C₁-C₄ éventuellement hydrogéné et/ou chloré ayant un point d'ébullition compris entre -39°C et +50°C;
- dans la demande de brevet EP 508 631 qui décrit la production de composés H.F.C. par réduction chimique en phase liquide de composés chlorés, bromés ou iodés avec un hydrure métallique ou un complexe d'un tel hydrure, il est indiqué que ce procédé peut être intéressant pour purifier certains H.F.C. comme le F125 ;
- récemment les demandes de brevet WO 94/02439 et WO 94/20441 proposent de purifier le F125 par hydrogénolyse des impuretés contenues (entre autres le F115) sur des catalyseurs métalliques déposés sur charbon, alumine ou alumine fluorée.

Parmi les différentes méthodes de purification proposées, l'hydrogénolyse présente l'avantage non négligeable d'épurer le F125 tout en valorisant l'impureté F115 à éliminer. Cependant, l'inconvénient majeur des procédé d'hydrogénolyse se situe au niveau de la stabilité dans le temps de l'activité catalytique. En effet, dans les conditions réactionnelles souvent sévères nécessaires à la transformation des réactifs, le catalyseur se désactive avec le temps ; il est alors nécessaire de le remplacer périodiquement par une charge neuve ou de trouver un moyen efficace de régénération du catalyseur usagé.

Dans cette optique, plusieurs techniques de régénération de catalyseurs d'hydrogénolyse se trouvent décrits dans la littérature. La demande de brevet WO 93/24224 propose l'oxydation du catalyseur usagé par l'oxygène ou un agent oxydant. Des traitements au chlore (brevet US 5 057 470) ou au C.F.C., qui peut être le réactif à transformer (brevet US 4 980 324), se révèlent également efficaces. Cependant, ces procédés ne font que réactiver les catalyseurs qui présentent toujours après le traitement les mêmes inconvénients.

Il a maintenant été trouvé que, contrairement aux autres catalyseurs communément employés : Pd/C, Pd/Al₂O₃ ou Pd/alumine fluorée, un catalyseur à base de palladium déposé sur du trifluorure d'aluminium (AlF₃) a la propriété d'être stable dans la réaction de purification du F125 par hydrogénolyse de l'impureté F115.

L'invention a donc pour objet un procédé de purification d'un pentafluoroéthane (F125) contenant du chloro-pentafluoroéthane (F115) par hydrogénolyse catalytique en phase vapeur, caractérisé en ce que l'on utilise un catalyseur à base de palladium déposé sur du trifluorure d'aluminium.

Le trifluorure d'aluminium est un support commercialement disponible dont la surface BET est généralement supérieure à 70 m²/g. Selon l'invention on l'utilise avantageusement sous forme de pastilles, mais on ne sortirait pas du cadre de la présente invention en utilisant d'autres formes telles que billes, extrudats...

Le catalyseur utilisé selon l'invention peut être préparé suivant les techniques classiques d'imprégnation du support au moyen d'une solution aqueuse ou organique d'un dérivé du palladium, en une quantité suffisante pour que la teneur pondérale en palladium du catalyseur soit comprise entre 0,1 et 10 %, de préférence entre 0,5 et 5 %. Après imprégnation, l'eau ou le solvant organique est éliminé par évaporation et le solide obtenu est soumis à un traitement thermique à une température allant de 100 à 500°C (de préférence 200 à 350°C) et sous courant d'hydrogène et/ou d'azote (de préférence sous une pression de 1 à 5 bars) pour libérer le palladium. La nature du dérivé du palladium est sans importance et peut être par exemple le chlorure, l'acétate, l'acétylacétonate, le nitrate ou les complexes organométalliques du chlorure. Le solvant peut être l'eau ou un composé organique.

Comme solvants organiques on peut utiliser les dérivés chlorés du méthane ou de l'éthane (par exemple le chloroforme, le chlorure de méthylène et le tétrachlorure de carbone), les solvants aromatiques (par exemple le benzène, le toluène et le chlorobenzène) ou des amines ou alcanolamines (par exemple la pyridine et l'éthanolamine).

La température du traitement de purification est généralement comprise entre 200 et 450°C, mais il est préférable de travailler entre 250 et 350°C. Le traitement peut être effectué à une pression comprise entre 1 et 50 bars; une augmentation de la pression augmente le temps de contact et permet d'atteindre des conversions plus élevées pour une température donnée.

Bien que le procédé selon l'invention puisse être appliqué à des mélanges contenant des teneurs élevées en F115, il est plus particulièrement destiné au traitement d'un F125 brut dont la teneur molaire en F115 ne dépasse pas 10 %. L'hydrogène est utilisé en une quantité telle que le rapport molaire H₂/F115 soit compris entre 1 et 40, de préférence entre 2 et 10.

Le débit horaire total de gaz (H₂ + F125 + F115) peut aller de 0,01 à 20 moles par litre de catalyseur et est de préférence compris entre 0,5 et 15 moles.

Les exemples suivants illustrent l'invention sans la limiter.

### EXEMPLE 1 Comparatif

Dans un réacteur tubulaire en Inconel de 45 cm de long et de 2,72 cm de diamètre intérieur, chauffé électriquement, on a introduit 50 ml d'un catalyseur commercial Pd/Al₂O₃ contenant 2 % en poids de palladium. On a fait passer sur le catalyseur un mélange d'hydrogène, de pentafluoroéthane (F125) et de chloropentafluoroéthane (F115) dans les conditions opératoires suivantes :
- Température: : 280°C
- Débit d'hydrogène: : 0,016 mole/heure
- Débit de F125: : 0,244 mole/heure
- Débit de F115: : 0,008 mole/heure

L'analyse, faite par chromatographie (CPV) en ligne à la sortie du réacteur, a donné les résultats suivants :

| **TEMPS (heures)** | **CONVERSION (%) du F115** | **SELECTIVITE (%) en F125** |
|---|---|---|
| 25 | 99,0 | 98,7 |
| 30 | 98,9 | 98,8 |
| 50 | 98,7 | 98,9 |
| 60 | 98,8 | 98,7 |
| 100 | 98,3 | 98,7 |
| 120 | 97,9 | 98,8 |
| 140 | 95,2 | 98,9 |
| 150 | 95,0 | 98,7 |
| 210 | 90,0 | 98,8 |
| 260 | 84,5 | 98,9 |

L'activité du catalyseur n'est pas stable dans le temps ; elle chute de 15 % environ après 260 heures de traitement continu.

### EXEMPLE 2 Comparatif

### a) Préparation du catalyseur (Pd/alumine fluorée)

Dans un réacteur tubulaire, on a chargé 75 ml (50 g) d'une alumine commerciale (180 m²/g) sous forme de billes (2 mm de diamètre) et on l'a séchée pendant 18 heures à 180°C sous courant d'air (1 mole/heure). On a ensuite monté la température de 180°C à 300°C en 5 heures avec un mélange d'air et d'acide fluorhydrique (2 moles/heure et 0,8 mole/heure respectivement), puis on a traité à 400°C sous HF pur (0,8 mole/heure) pendant 8 heures. On a ainsi obtenu une alumine fluorée contenant 64,5 % massique de fluor et ayant une surface BET de 20 m²/g.

Dans un évaporateur rotatif, on a chargé 50 ml (28 g) de l'alumine fluorée préparée de la manière décrite ci-dessus. Après dégazage pendant 3 heures à 100°C sous pression réduite (1 kPa), on a introduit 50 ml d'une solution d'acétate de palladium dans le toluène contenant 0,6 g de palladium, puis on a évaporé le solvant sous pression réduite (26 kPa) et séché à 80°C. On a traité ensuite à 250°C pendant 2 heures sous un courant d'azote (5 Nl/h) et obtenu ainsi un catalyseur Pd/alumine fluorée contenant 2,3 % de palladium.

### b) Purification du F125

Dans un réacteur tubulaire en Inconel de 45 cm de long et de 2,72 cm de diamètre intérieur, chauffé électriquement, on a introduit 50 ml du catalyseur dont la préparation est décrite ci-dessus, puis on a fait passer sur le catalyseur un mélange d'hydrogène, de pentafluoroéthane (F125) et de chloropentafluoroéthane (F115) dans les conditions opératoires suivantes :
- Température: : 270°C
- Débit d'hydrogène: : 0,018 mole/heure
- Débit de F125: : 0,271 mole/heure
- Débit de F115: : 0,009 mole/heure

L'analyse, faite par chromatographie (CPV) en ligne à la sortie du réacteur, a donné les résultats suivants :

| **TEMPS (heures)** | **CONVERSION (%) du F115** | **SELECTIVITE (%) en F125** |
|---|---|---|
| 10 | 70,4 | 97,9 |
| 20 | 60,8 | 97,7 |
| 25 | 53,9 | 97,1 |
| 30 | 48,3 | 96,9 |
| 40 | 32,7 | 96,2 |
| 50 | 22,8 | 94,4 |
| 60 | 17,3 | 91,9 |
| 70 | 14,7 | 91,0 |
| 90 | 13,1 | 90,5 |

L'activité du catalyseur n'est pas stable dans le temps.

### EXEMPLE 3

### a) Préparation du catalyseur (Pd/trifluorure d'aluminium)

Dans un évaporateur rotatif, on a chargé 50 ml (36 g) de fluorure d'aluminium commercial ayant une surface BET de 110 m²/g, préalablement mis en forme de pastilles (5 mm de diamètre sur 3 mm de hauteur). Après dégazage pendant 3 heures à 100°C sous pression réduite (1 kPa), on a introduit 50 ml d'une solution d'acétate de palladium dans le toluène contenant 1 g de palladium, puis on a évaporé le solvant sous pression réduite (26 kPa) et séché à 80°C. On a traité ensuite à 250°C pendant 2 heures sous un courant d'azote (5 Nl/h) et obtenu ainsi un catalyseur Pd/trifluorure d'aluminium contenant 2,4 % de palladium.

### b) Purification du F125

Dans un réacteur tubulaire en Inconel de 45 cm de long et de 2,72 cm de diamètre intérieur, chauffé électriquement, on a introduit 43 ml du catalyseur dont la préparation est décrite ci-dessus, puis on a fait passer sur le catalyseur un mélange d'hydrogène, de pentafluoroéthane (F125) et de chloropentafluoroéthane (F115) dans les conditions opératoires suivantes :
- Température: : 290°C
- Débit d'hydrogène: : 0,018 mole/heure
- Débit de F125: : 0,225 mole/heure
- Débit de F115: : 0,009 mole/heure

L'analyse, faite par chromatographie (CPV) en ligne à la sortie du réacteur, a donné les résultats suivants :

| **TEMPS (heures)** | **CONVERSION (%) du F115** | **SELECTIVITE (%) en F125** |
|---|---|---|
| 10 | 98,5 | 98,4 |
| 50 | 98,8 | 98,3 |
| 70 | 98,5 | 98,5 |
| 140 | 98,6 | 98,6 |
| 150 | 98,7 | 98,8 |
| 160 | 98,8 | 98,9 |
| 180 | 98,6 | 98,8 |
| 230 | 98,6 | 98,7 |
| 250 | 98,6 | 98,9 |
| 320 | 98,6 | 99,0 |
| 350 | 98,5 | 98,8 |
| 400 | 98,6 | 98,9 |

Le catalyseur est parfaitement stable.

## Revendications

1. Procédé de purification d'un pentafluoroéthane brut contenant du chloropentafluoroéthane par hydrogénolyse catalytique en phase vapeur, caractérisé en ce que l'on utilise un catalyseur à base de palladium déposé sur du trifluorure d'aluminium.

2. Procédé selon la revendication 1, dans lequel le trifluorure d'aluminium a une surface BET supérieure à 70 m²/g.

3. Procédé selon la revendication 1 ou 2, dans lequel la teneur pondérale en palladium du catalyseur est comprise entre 0,1 et 10 %, de préférence entre 0,5 et 5 %.

4. Procédé selon l'une des revendications 1 à 3, dans lequel on opère à une température comprise entre 200 et 450°C, de préférence entre 250 et 350°C.

5. Procédé selon l'une des revendications 1 à 4, dans lequel on opère à une pression comprise entre 1 et 50 bars.

6. Procédé selon l'une des revendications 1 à 5, dans lequel le rapport molaire hydrogène/chloropentafluoroéthane est compris entre 1 et 40, de préférence entre 2 et 10.

7. Procédé selon l'une des revendications 1 à 6, dans lequel le débit total de gaz par litre de catalyseur est compris entre 0,01 et 20 moles, de préférence entre 0,5 et 15 moles.

8. Procédé selon l'une des revendications 1 à 7, dans lequel la teneur molaire en chloropentafluoroéthane du pentafluoroéthane brut à traiter n'excède pas 10 %.

## Claims

1. Process for the purification of a crude pentafluoroethane comprising chloropentafluoroethane by vapour-phase catalytic hydrogenolysis, characterized in that use is made of a catalyst based on palladium deposited on aluminium trifluoride.

2. Process according to Claim 1, in which the aluminium trifluoride has a BET surface of greater than 70 m²/g.

3. Process according to Claim 1 or 2, in which the content by weight of palladium in the catalyst is between 0.1 and 10%, preferably between 0.5 and 5%.

4. Process according to one of Claims 1 to 3, in which the reaction is carried out at a temperature of between 200 and 450°C, preferably between 250 and 350°C.

5. Process according to one of Claims 1 to 4, in which the reaction is carried out at a pressure of between 1 and 50 bar.

6. Process according to one of Claims 1 to 5, in which the hydrogen/chloropentafluoroethane molar ratio is between 1 and 40, preferably between 2 and 10.

7. Process according to one of Claims 1 to 6, in which the total throughput of gas per litre of catalyst is between 0.01 and 20 mol, preferably between 0.5 and 15 mol.

8. Process according to one of Claims 1 to 7, in which the molar content of chloropentafluoroethane in the crude pentafluoroethane to be treated does not exceed 10%.

## Patentansprüche

1. Verfahren zur Reinigung von rohem Pentafluorethan, das Chlorpentafluorethan enthält, durch katalytische Hydrogenolyse in Gasphase, dadurch gekennzeichnet, daß man einen Katalysator auf Basis von Palladium enthaltendem Aluminiumtrifluorid verwendet.

2. Verfahren nach Anspruch 1, in dem das Aluminiumtrifluorid eine BET-Oberfläche von mehr als 70 m²/g aufweist.

3. Verfahren nach Anspruch 1 oder 2, in dem der Gewichtsanteil des Palladiums im Katalysator zwischen 0,1 und 10 %, vorzugsweise zwischen 0,5 und 5 %, liegt.

4. Verfahren nach einem der Ansprüche 1 bis 3, in dem man bei einer Temperatur zwischen 200 und 450 °C, vorzugsweise zwischen 250 und 350 °C, arbeitet.

5. Verfahren nach einem der Ansprüche 1 bis 4, in dem man bei einem Druck zwischen 1 und 50 bar arbeitet.

6. Verfahren nach einem der Ansprüche 1 bis 5, in dem das Molverhältnis Wasserstoff/Chlorpentafluorethan zwischen 1 und 40, vorzugsweise zwischen 2 und 10, liegt.

7. Verfahren nach einem der Ansprüche 1 bis 6, in dem die Gesamtmenge an Gas je Liter des Katalysators zwischen 0,01 und 20 Mol, vorzugsweise zwischen 0,5 und 15 Mol, liegt.

8. Verfahren nach einem der Ansprüche 1 bis 7, in dem der Stoffmengenanteil von Chlorpentafluorethan im zu behandelnden rohen Pentafluorethan 10 % nicht überschreitet.
